# EUROPÄISCHE PATENTSCHRIFT

(11) **EP 2 520 314 B1**
(45) Veröffentlichungstag und Bekanntmachung des Hinweises auf die Patenterteilung: **02.09.2015**
(21) Anmeldenummer: 12166414.8
(22) Anmeldetag: 02.05.2012
(51) Int. Cl.: A61L 2/26, A61B 19/02

(54) **Filtereinheit**
Filter unit
Unité de filtre

(30) Priorität: 02.05.2011 DE 102011050029; 17.08.2011 DE 102011052789; 13.10.2011 DE 102011054469; 14.03.2012 DE 102012102167
(43) Veröffentlichungstag der Anmeldung: 07.11.2012
(73) Patentinhaber: Ermis, Mehmet, 78532 Tuttlingen (DE)
(72) Erfinder: Ermis, Mehmet, 78532 Tuttlingen (DE)
(74) Vertreter: Kuhnen & Wacker

(56) Entgegenhaltungen:
- DE-U1-202007 004 639
- US-A- 4 783 321
- US-A- 6 077 485
- US-A1- 2004 256 268

## Beschreibung

Die Erfindung betrifft einen Sterilisierbehälter nach dem Anspruch 1 und eine Verwendung nach dem Anspruch 10.

### Stand der Technik

Aus dem Stand der Technik sind verschiedene Arten von Sterilisierbehältern bekannt. Hierbei wird zunächst auf die DE 2 610 290 A1 hingewiesen. Dort ist ein Sterilisierbehälter mit einem Ventilfilter oder Ventilverschluss beschrieben. Weiter wird auf die von der Firma Gebrüder Martin GmbH als Sterilbarriere vertriebene Anordnung hingewiesen.

Die DE 20 2007 004 639 U1 zeigt eine Filterhalterung für einen Sterilbehälter mit einem Filterträger mit einer ersten Verbindungseinrichtung zum Verbinden der Filterhalterung mit dem Sterilbehälter und mit einer zweiten Verbindungseinrichtung zum Verbinden der Filterhalterung mit einem Filter, wobei die zweite Verbindungseinrichtung ein zentral mittig vom Filterträger abstehendes Verbindungselement aufweist.

Die US 4.783,321 zeigt ein Sterilisationscontainersystem mit einem über Haltebügel mit einem Bodenteil verbundenen Deckel. Auswechselbare Papierfilter sind über Öffnungen auf der Innenseite des Deckels abgedichtet, um die Sterilität des Inhalts des Sterilisationscontainersystems nach der Sterilisation aufrecht zu erhalten. Eine zweiteilige scheibenförmige Papierfilteraufnahmevorrichtung mit nicht ausgerichteten Öffnungen verhindertein ein Einreißen oder Durchstechen der Papierfilter durch die zu sterilisierenden Instrumente.

Die US 2004/0256268 A1 zeigt einen Sterilisationscontainer mit einem Aufnahmebereich, der durch einen Containerboden und Containerwände gebildet wird, einen Deckel zum Schließen des Aufnahmebereichs, und einer Gasaustauschöffnung, die mittels einem sterilen Filter, der in einem Filterhalter angeordnet ist, abgedichtet ist. Zum einfachen Auswechseln des sterilen Filters und um die Produktion des Deckels zu vereinfachen bilden der sterile Filter und der Filterhalter eine Filtereinheit, die an den Deckel angebracht werden kann.

Zuletzt wird auf die DE 20 2004 006 725 U1 verwiesen, welche unter anderem den Einsatz eines Teflonfilters offenbart.

Die US 6,077,485 zeigt außerdem einen Aufbewahrungsbehälter mit einem Behälterabschnitt und einem Deckel, die mit Hilfe von einem Paar federnd gelagerter Endhebeln sicher miteinander verbunden werden können. Der Deckel umfasst unter anderem ein Paar flacher Filteranordnungen. Jede Filteranordnung enthält unter anderem eine Gehäuseplatte, die mittels schlüssellochartiger Ausbuchtungen in der Gehäuseplatte und in dem Deckel angeordneter Verschlussbolzen einfach befestigt werden können.

### Aufgabe

Aufgabe der Erfindung ist es einen Deckel für einen Sterilisierbehälter zu schaffen, welcher mit unterschiedlichsten Arten von Filtern betrieben werden kann. Hierbei soll ein und derselbe Deckel nach den Bedürfnissen des Nutzers oder rechtlichen Vorgaben mit einer bevorzugten Art von Filter, wie Teflonfilter oder Ventilfilter oder Sterilbarrieren auf möglichst einfache Weise bestückbar sein.

### Lösung der Aufgabe

Zur Lösung der Aufgabe führt Anspruch 1.

Eine erfindungsgemässer Deckel für einen Sterilisierbehälter ist in der Regel aus Aluminium hergestellt. Denkbar sind aber auch Sterilisierbehälterdeckel aus Kunststoff oder Chrom/Nickel. Im Ergebnis ist darauf zu achten, dass das genutzte Material den Anforderungen einer Sterilisierung bezüglich Temperatur, Druck und Dauer ausreichend Stand hält. Bei Erfüllen dieser Kriterien kommt praktisch jedes Material für den Sterilisierbehälter in Frage. Vorteilhaft gerade an metallischen Deckeln ist aber der Umstand, dass Sie zum Einen sehr schnell die Wärme aufnehmen und zum Anderen die Wärme sehr schnell wieder abgeben. Dies ist für den alltäglichen Gebrauch von Vorteil, da das Nutzerpersonal entsprechend kein Schutzhandschuhe benötigt.

Ein erfindungsgemässer Deckel weist ausserdem ein Aufnahmegehäuse auf. Dieses Aufnahmegehäuse ist derart gestaltet, dass ein Filter integriert ist. Dies bedeutet, dass das Aufnahmegehäuse in sich abgeschlossen ausgeführt ist, sodass der Filter in dem Aufnahmegehäuse ausgeliefert wird. Bei dem Filter kann es sich um handelsübliche Filter, wie Sterilbarrieren oder Ventilfilter oder Teflonfilter handeln. Das Aufnahmegehäuse ist so zu gestalten, dass die verschiedenen Filterformen ohne weiteres integriert werden können. Das Material des Aufnahmegehäuses ist bevorzugt aus Kunststoff, wobei aber auch andere Materialien wie Aluminium oder Chrom/Nickel zum Einsatz kommen können. Auch hier ist letztendlich nur von Belang, ob die eingesetzten Materialen den Erfordernissen bezüglich Temperatur, Druck und Dauer ausreichend Stand halten, damit eine Sterilisierung der chirurgischen Instrumente erfolgen kann.

Ein erfindungsgemässer Deckel weist ausserdem einen Universalverschluss auf, welcher die wiederlösbare Verbindung des Aufnahmegehäuses mit dem Deckel ermöglicht. Dieser Universalverschluss ist derart gestaltet, dass durch einen einfachen Handgriff, wie Ansetzen und Drehen oder Aufdrücken eine wiederlösbare Verbindung geschaffen wird. Diese wiederlösbare Verbindung ist in der Weise von Vorteil, dass verschiedenste Arten von Filtern, welche in dem Aufnahmegehäuse integriert sind an einen Standarddeckel mit dem entsprechenden Universalverschluss verbunden und wieder gelöst werden können. Dies hat den Vorteil, dass der Nutzer beispielsweise je nach den Bedürfnissen eines Bestellers das vorgefertigte Aufnahmegehäuse mit dem integrierten Filter mit dem Deckel verbinden kann. Diese Modulbauweise erspart es dem Nutzer verschiedenste Arten von Deckeln mit integriertem Filter auf Lager vorrätig zu halten. Vielmehr kann er nun einen Deckel mit einem entsprechenden Festlegeprofil lagern und unterschiedlichste Arten von Filtern mit dem Deckel verbinden, in dem er das Aufnahmegehäuse über den Universalverschluss wiederlösbar mit dem Deckel verbindet.

In einem bevorzugten Ausführungsbeispiel weist das Aufnahmegehäuse auf der dem Deckel zugewandten Seite eine teilweise Hinterschneidung auf, welche dazu dient mit entsprechenden Hakenelementen des Festlegeprofils zusammenzuwirken. Dies bedeutet letztendlich, dass der Nutzer das Aufnahmegehäuse beispielsweise auf die Unterseite des Deckels aufsetzt und die Hakenelemente des Festlegeprofils zunächst in die nicht festlegenden Bereiche des Aufnahmegehäuses anlegt um anschliessend durch eine Drehbewegung, ähnlich einem Bajonettverschluss, die Hakenelemente des Festlegeprofils mit den Hinterschneidungen des Aufnahmegehäuses in Wirkverbindung zu bringen. Dabei weisen die Hinterschneidungen ausserdem einen Anschlag auf, sodass die Drehbewegung an dem Punkt gestoppt wird, an dem die Hakenelemente an dem Ausschlag der Hinterschneidung anstossen. Dies hat den Vorteil, dass der Nutzer schnell und einfach ein entsprechendes Aufnahmegehäuse mit integrierten Filter mit einem Deckel verbinden kann. Hierbei handelt es sich lediglich um ein Ausführungsbeispiel von anderen. Dieses Ausführungsbeispiel weist sich lediglich durch eine einfache Herstellung und Handhabbarkeit aus. Denkbar wären ebenfalls ein Gewinde, welches als Festlegeprofil auf der Unterseite des Deckels angebracht ist, wobei das Aufnahmegehäuse dann ein mit diesem Gewinde korrespondierendes Gegenelement aufweisen müsste, sodass der Nutzer das Aufnahmegehäuse an den Deckel anschraubt. In diesem Zusammenhang ist lediglich von Belang, dass der Universalverschluss derart gestaltet ist, dass er wiederlösbar ist. Das bedeutet, dass der Nutzer ohne Einsatz von Werkzeug das Aufnahmegehäuse mit dem Deckel verbinden und wieder lösen können soll. Das Lösen des Aufnahmegehäuses vom Deckel soll dabei ohne Beeinträchtigung von Material geschehen. Das Lösen ohne Werkzeug wiederum bedeutet, dass der Nutzer lediglich durch Einsatz von manueller Kraft und seinen Händen das Aufnahmegehäuse mit dem Deckel verbinden oder lösen können soll.

Ein erfindungsgemässes Ausführungsbeispiel des Aufnahmegehäuses weist ausserdem auf der dem Deckel zugewandten Seite einen Dichtungsring auf. Dieser Dichtungsring erfüllt die Aufgabe eines luftdichten Verschlusses zwischen dem Aufnahmegehäuse und dem Deckel. Dies wiederum gewährleistet den Umstand, dass kein Sterilisiermedium ungewollt in den Sterilisierbehälter oder aus dem Sterilisierbehälter entweichen kann. Jegliches Sterilisiermedium, welches in den Sterilisierbehälter gelangt, muss durch den entsprechend im Aufnahmegehäuse integrierten Filter geführt werden. In gleicher Weise soll das im Sterilisierbehälter vorhandene Sterilisiermedium ausschliesslich durch den Filter wieder entweichen können. Das Aufnahmegehäuse bietet hierzu die Möglichkeit des Durchflusses solcher geeigneter Medien zur Sterilisierung von chirurgischen Instrumenten.

Ein weiteres erfindungsgemässes Ausführungsbeispiel weist auf der dem Deckel abgewandten Seite eine erste Festlege-Löse-Markierung auf. Diese Festlege-Löse-Markierung dient dazu, anhand einer zweiten Festlege-Löse-Markierung, welche auf dem Deckel angebracht ist, zu korrespondieren. Hierbei ist die zweite Festlege-Löse-Markierung auf der dem Aufnahmegehäuse zugewandten Seite angebracht. Die Einzelheiten einer solchen Festlege-Löse-Markierung sind unbeachtlich, solange auf dem Aufnahmegehäuse und auf dem Deckel jeweils an der oben beschriebenen Stelle eine solche erste und zweite Festlege-Löse-Markierung vorhanden ist. Dies hat den Vorteil, dass der Nutzer auf einfache Art und Weise feststellen kann, an welchem Punkt er das Aufnahmegehäuse an den Deckel ansetzen muss, ohne dass das Festlegeprofil hierbei behindernd wirkt. Nach dem Aufsetzen des Aufnahmegehäuses auf den Deckel kann der Nutzer dann anhand von aufgebrachten Pfeilen und der Markierung auf dem Deckel erkennen, in welcher Richtung er das Aufnahmegehäuse drehen soll, um einen Verschluss und damit eine Verbindung zwischen Aufnahmegehäuse und Deckel zu schaffen. In gleicher Weise kann er umgekehrt das Aufnahmegehäuse vom Deckel wieder lösen.

Ausserdem soll gesondert Schutz für eine elektrische Markierung eines Sterilisierbehälters mithilfe eines Empfänger- oder Senderchips begehrt werden. Hierzu wird ein Chip an einem Teil des Sterilisierbehälters integriert. Ein solcher Chip kann daher im Aufnahmegehäuse und/oder dem Deckel integriert sein.

Es kann sich hierbei grundsätzlich um jede Art von Chip handeln. Beispielsweise kommen so genannte RFID-Chips ebenso in Betracht, wie auch andere Datenträger, welche an vorgesehenen Stationen durch Leseeinheiten ausgelesen werden können. Die elektrische Markierung kann neben einem Chip oder einer Empfänger-oder Sendereinheit auch einen GPS-Sensor umfassen, welcher die lückenlose Überwachung des Sterilisierbehälters gewährleistet.

Im Rahmen der eingesetzten Chips können auch Daten über Nutzungsintervalle, Temperatur, Temperaturschwankungen oder Standort des Sterilisierbehälters erfasst und wiedergegeben werden. Hierzu kann der eingesetzte Chip auch über Sensoren verfügen, welche beispielsweise den Temperaturverlauf aufzeichnen und wiedergeben können.

Die Markierung wird mit so genannten Lasereinheiten zusammen. Dies bedeutet im einzelnen, dass Leseeinheiten beispielsweise an Ausgängen von Räumen oder an Aus- oder Einfahrten von Sterilisiervorrichtungen angebracht sein können. Aufgrund des Zusammenwirkens zwischen der Markierung und der Leseeinheit kann lückenlos ermittelt werden, wann, wo und wie sich der Sterilisierbehälter befunden hat.

### FIGURENBESCHREIBUNG

Weitere Vorteile, Merkmale und Einzelheiten der Erfindung werden in den nachfolgend gezeigten Figuren beschrieben. Diese zeigt in
- Figur 1: eine geschnittene Seitenansicht eines Sterilisierbehälters;
- Figur 2: eine Draufsicht auf einen erfindungsgemässen Deckel;
- Figur 3: eine Draufsicht auf ein erfindungsgemässes Aufnahmegehäuse;
- Figur 4: eine vergrösserte Teilansicht der Figur 3;
- Figur 5: eine geschnittene Seitenansicht eines erfindungsgemässen Aufnahmegehäuses nach Figur 3;
- Figur 6: eine Draufsicht auf einen erfindungsgemässen Deckel;
- Figur 7.: eine Draufsicht auf ein anderes Ausführungsbeispiel eines erfindungsgemässen Deckels;
- Figur 8: eine Draufsicht auf ein anderes Ausführungsbeispiel eines erfindungsgemässen Aufnahmegehäuses;
- Figur 9 Figur 8;: eine geschnittene Seitenansicht an der Schnittlinie A-A nach der
- Figur 10: eine Seitenansicht von schräg oben auf das Aufnahmegehäuse nach Figur 8;
- Figur 11 Figur 8;: eine Ansicht von schräg unten auf das Aufnahmegehäuse nach
- Figur 12: eine Draufsicht auf ein anderes Ausführungsbeispiels eines erfindungsgemässen Aufnahmegehäuses;
- Figur 13: eine geschnittene Seitenansicht an der Schnittlinie A-A nach der Figur 12;
- Figur 14: eine Draufsicht auf das Aufnahmegehäuse nach Figur 12 von schräg oben;
- Figur 15: eine Ansicht des Aufnahmegehäuses nach Figur 12 von schräg unten;
- Figur 16: eine Draufsicht auf ein weiteres erfindungsgemässes Ausführungsbeispiels eines erfindungsgemässen Aufnahmegehäuses;
- Figur 17: eine geschnittene Seitenansicht an der Schnittlinie A-A nach Figur 16;
- Figur 18: eine Ansicht von schräg oben auf das Ausführungsbeispiel gemäss Figur 16;
- Figur 19: eine Ansicht von schräg unten auf das Ausführungsbeispiel nach Figur 16.

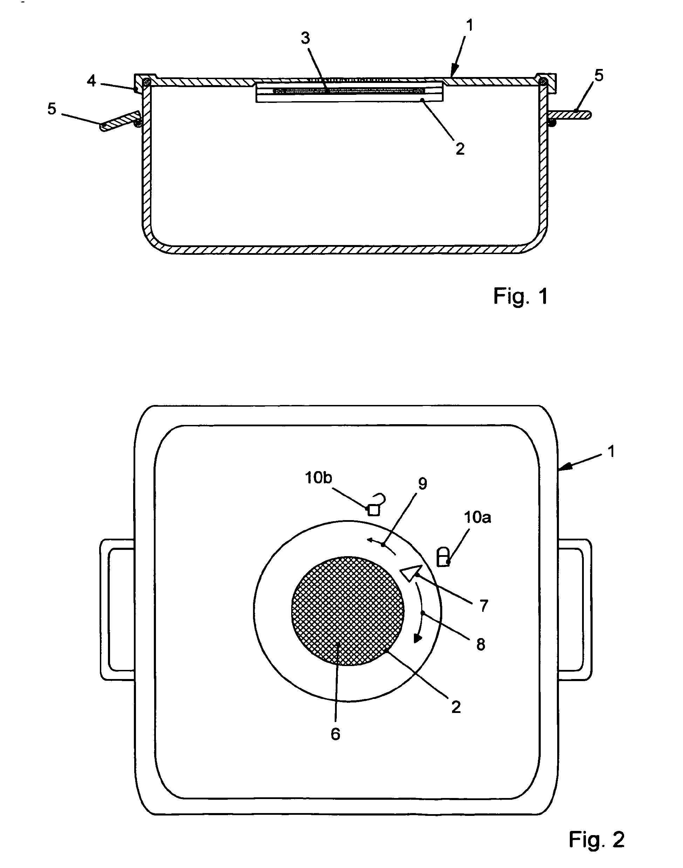

In Figur 1 ist zunächst eine geschnittene Seitenansicht eines wannenförmigen Sterilisierbehälters S gezeigt. Der Sterilisierbehälter S ist mit einem Deckel 1 verschlossen. Der Verschluss 4 sorgt hierbei dafür, dass der Deckel 1 mit dem Sterilisierbehälter S eine feste Verbindung eingeht. Ausserdem weist der Sterilisierbehälter S einen Henkel 5 zum besseren Transport des Sterilisierbehälters S auf. Daneben ist in der geschnittenen Seitenansicht auch auf der Unterseite des Deckels 1 ein Aufnahmegehäuse mit einem integrierten Filter 3 zu erkennen. Unterseite des Deckels bedeutet in diesem Zusammenhang die Seite des Deckels, welche dem Sterilisierbehälter S zugewandt ist.

In Figur 2 wird nun wiederum die Unterseite des Deckels 1 gezeigt. Auf der Unterseite des Deckels 1 ist zunächst wiederum das Aufnahmegehäuse 2 in verbundener Lage mit dem Deckel 1 gezeigt. Ausserdem ist eine Öffnung 6 zu erkennen, welche den Durchtritt des Sterilisiermediums durch das Aufnahmegehäuse 2 und den nicht gezeigten Filter 3 ermöglicht. Dass das Aufnahmegehäuse 2 in Figur 2 fest mit dem Deckel 1 verbunden ist, lässt sich daran erkennen, dass eine erste Festlege-Löse-Markierung 7 auf einer zweiten Festlege-Löse-Markierung 10a ruht. Die erste Festlege-Löse-Markierung 10a zeigt ein Symbol für "geschlossen". Die zweite Festlege-Löse-Markierung 10b wiederum zeigt ein Symbol für den Zustand "geöffnet". Wenn die erste Festlege-Löse-Markierung 7 anhand eines Pfeils 9 gedreht wird, so ist bei Erreichen der zweiten Festlege-Löse-Markierung 10b das Aufnahmegehäuse 2 vom Deckel 1 gelöst und somit abnehmbar. Beim Aufsetzen und Schliessen des Aufnahmegehäuses 2 mit dem Deckel 1 wird wiederum das Aufnahmegehäuse 2 zunächst auf die zweite Festlege-Löse-Markierung 10b aufgesetzt und dann anhand eines Pfeils 8 gedreht, sodass die erste Festlege-Löse-Markierung 7 auf die erste Festlege-Löse-Markierung 10a gedreht wird. Dabei wird das Aufnahmegehäuse 2 mit dem Deckel 1 wiederlösbar verbunden.

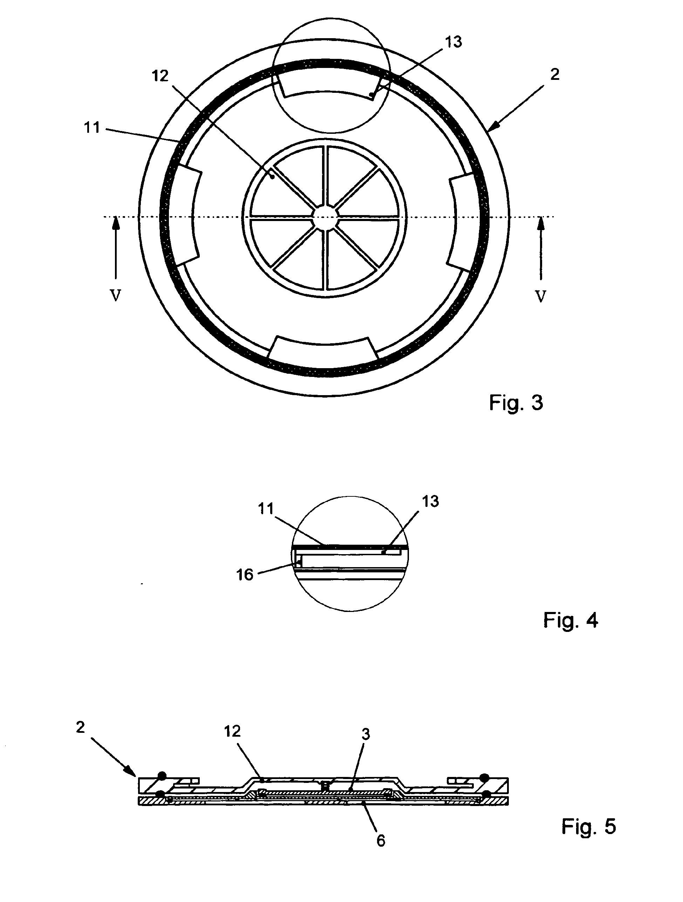

In der Figur 3 wiederum ist lediglich eine Draufsicht auf das Aufnahmegehäuse 2 gezeigt. Die Draufsicht aus Figur 3 zeigt die Seite des Aufnahmegehäuses 2, welche beim Verbinden mit dem Deckel 1, dem Deckel 1 zugewandt ist. Dort ist ein Dichtungsring 11 gezeigt, welcher beim Verbinden des Aufnahmegehäuses 2 mit dem Deckel 1 einen luftdichten Abschluss ermöglicht, sodass das Sterilisiermedium durch eine Öffnung 12 geführt wird. Die Öffnung 12 wiederum korrespondiert mit der Öffnung 6, welche in der Figur 3 nicht gezeigt ist. Bei Durchschreiten des Sterilisiermediums durch die Öffnung 12 wird zunächst der Filter 3 und anschliessend die Öffnung 6 im Inneren des Sterilisierbehälters durchflossen. Anschliessend werden die chirurgischen Instrumente im Innern des Sterilisierbehälters S entsprechend desinfiziert und sterilisiert. Hierzu wird der Deckel 1 unter Druck mit Sterilisiermedium beaufschlagt. Ausserdem ist in Figur 3 eine Hinterschneidung 13 gezeigt. Diese Hinterschneidung 13 dient dem einfachen Festlegen des Aufnahmegehäuses 2 mit dem Deckel 1. Ausserdem ist eine Schnittlinie V eingezeichnet, welche zur besseren Erläuterung der Figur 3 notwendig ist.

In diesem Zusammenhang wird in Figur 4 eine vergrösserte Seitenansicht einer solchen Hinterschneidung 13 gezeigt. Hierbei ist sehr vereinfacht dargestellt, wie die Hinterschneidung 13 einen Anschlag 16 umfasst. Dieser Anschlag 16 dient dazu, der Drehbewegung des Aufnahmegehäuses 2 zum Festlegen an dem Deckel 1 einen Endpunkt zur Verfügung zu stellen.

Figur 5 zeigt wiederum eine auf der Schnittlinie V geschnittene Seitenansicht des Aufnahmegehäuses 2. Dort ist gut zu erkennen, wie einerseits die Öffnung 12 und andererseits die Öffnung 6 und zwischen den beiden Öffnungen 6, 12 der Filter 3 vorhanden ist.

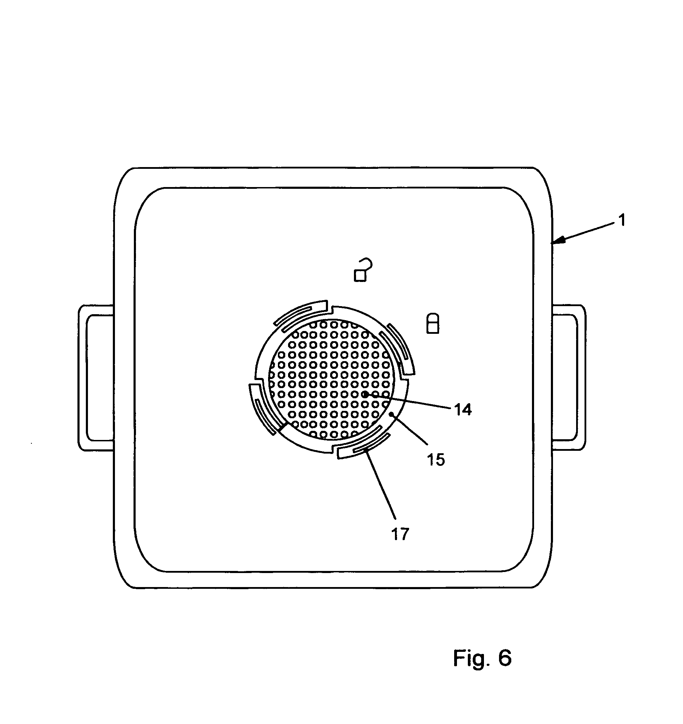

In Figur 6 ist die gleiche Ansicht, wie aus Figur 2 gezeigt. Allerdings ist in Figur 6 lediglich der Deckel 1 und nicht das an dem Deckel 1 angebrachte Aufnahmegehäuse 2. Dafür ist nun aber ein Festlegeprofil 15 zu erkennen. Dieses Festlegeprofil 15 weist ein Hakenelement 17 auf. Dieses Hakenelement 17 wirkt mit dem aus Figur 4 bekannten Anschlag 16 zusammen. Ausserdem ist in Figur 6 ein Durchbruch 14 zu erkennen. Dieser Durchbruch 14 gewährleistet, dass das mit Druck auf die andere Seite des Deckels 1 beaufschlagte Sterilisiermedium in das Innere des Sterilisierbehälters S gedrückt wird. Dabei durchläuft das Sterilisiermedium zunächst die Oberseite des Deckels 1, welche dem Sterilisierbehälter S abgewandt ist, um anschliessend durch die Öffnung 12, den Filter 3 und die Öffnung 6 in das Innere des Sterilisierbehälters S zu gelangen. Dort sterilisiert das Sterilisiermedium die entsprechend eingelegten chirurgischen Instrumente.

In diesem Zusammenhang soll darauf hingewiesen werden, dass entweder der Deckel 1 oder das Aufnahmegehäuse 2 über eine nicht näher gezeigte elektrische Markierung verfügt, welche über Sensoren Informationen sammelt und bei Abfrage durch eine Leseeinheit, welche auch portabel sein kann, die entsprechend gesammelten Informationen an die Leseeinheit abgibt.

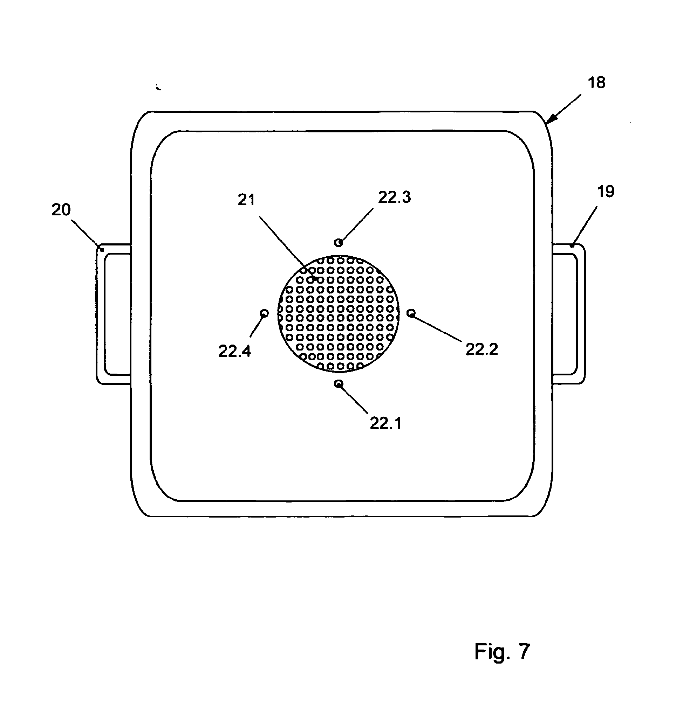

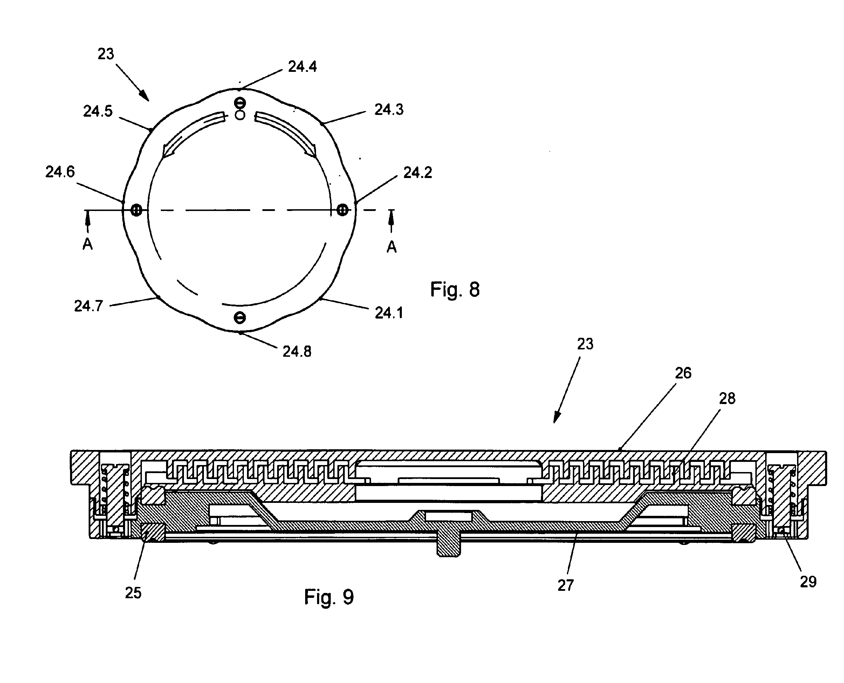

Figur 7 zeigt eine Draufsicht auf einen erfindungsgemässen Deckel 18. Dieser Deckel 18 weist zwei Festlegeprofile 19, 20 auf, welche dazu dienen, den Deckel auf einem nicht gezeigten Aufnahmegehäuse festzulegen. Im mittigen Bereich ist ein Durchbruch 21 mit einer Vielzahl von Öffnungen gezeigt. Ausserdem sind vier festsitzende Schraubenelemente 22.1 bis 22.4 gezeigt. Diese Schraubenelemente durchgreifen den Deckel 18 und dienen andernseits des Deckels 18 zur festen Verbindung des Deckels mit einer zusätzlichen Plastikhaube über dem Durchbruch 21, welcher in Figur 7 nicht zu erkennen ist. Ausserdem sind die Schraubenelemente 22.1 bis 22.4 derart gestaltet, dass ein Aufnahmegehäuse 23 nach Figur 8 durch Aufsetzen und Drehen, ähnliche einem Bajonettverschluss, an den Schraubenelementen 22.1 bis 22.4 festgelegt werden kann. Das Aufnahmegehäuse 23 weist ausserdem verschiedene Ausbuchtungen 24.1 bis 24.8 auf.

In Figur 9 ist nun gut zu erkennen, dass es sich bei dem Ausführungsbeispiel nach Figur 8 um die Filtertechnologie nach dem sogenannten Barrieresystem handelt. Das Aufnahmegehäuse 23 besteht aus einer Silikondichtung 25, welche auf der Unterseite des Aufnahmegehäuses 23 angebracht ist. In gleicher Weise ist auch gezeigt, wie ein Deckelteil 26 mit einem Unterteil 27 derart verbunden ist, dass im Inneren ein labyrinthartiges Barrieresystem 28 entsteht.

Das Aufnahmegehäuse 23 weist ausserdem eine federgelagerte Kugel 29 auf, welche beim Verbinden mit dem Deckel 18 in nichtgezeigte aber vorhandene und vorgesehene kugelförmige Ausbuchtungen des Deckels 18 eingreift, wenn das Aufnahmegehäuse 23 an der richtigen Position festgelegt werden kann.

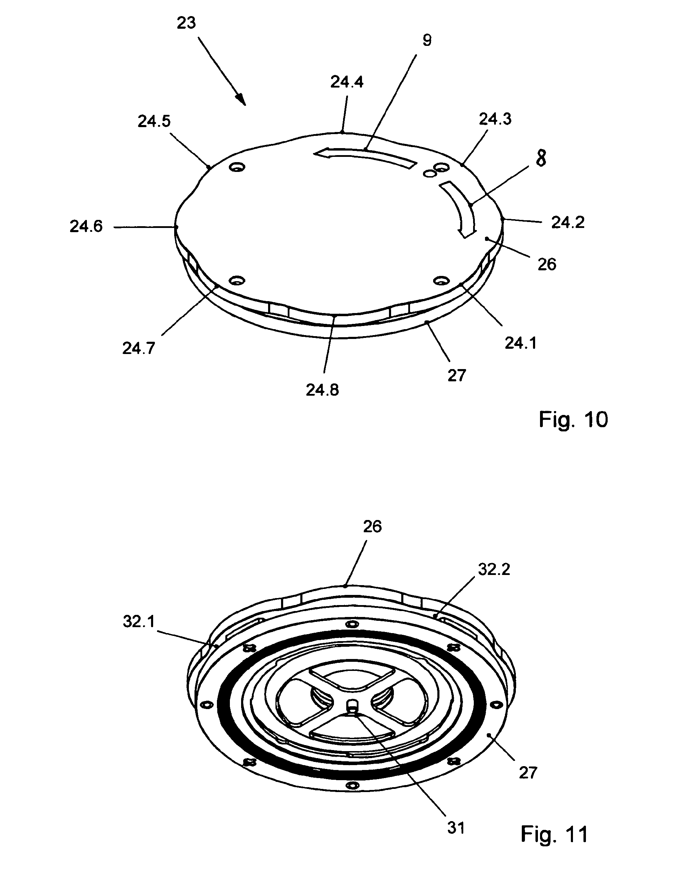

Figur 10 zeigt nochmals eine Draufsicht eines erfindungsgemässen Aufnahmegehäuses 23, sowie die Ausbuchtungen 24.1 bis 24.8. Ausserdem ist in Figur 10 gezeigt, wie auf dem Deckelteil 26 zwei Dichtungspfeile 30.1, 30.2 eingelassen bzw. aufgebracht sind. Daneben ist auch das Unterteil 27 noch als gut im Zusammenspiel mit dem Deckelteil 26 gezeigt.

Figur 11 zeigt nun eine Ansicht von schräg unten auf das Aufnahmegehäuse nach Figur 8. Dort ist wiederum zu erkennen, wie das Unterteil 27 einen Einlass 31 aufweist, durch den die Luft, welche durch die Öffnung im Durchbruch 21 in das Innere des Sterilisierbehälters einfliessen soll, durch das hier nicht gezeigte Barrieresystem an den Auslässen 32.1, 32.2 in das Innere des nicht gezeigten Sterilisierbehälters einfliessen soll.

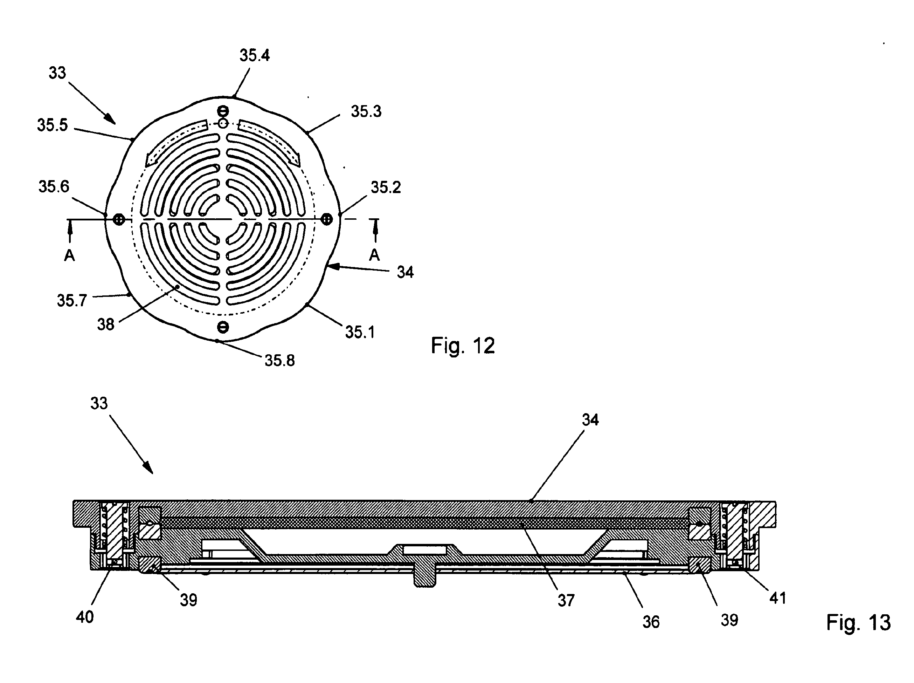

Figur 12 zeigt ein weiteres Ausführungsbeispiel eines erfindungsgemässen Gehäuses 33. Auch dieses Aufnahmegehäuse 33. In Figur 12 ist Deckelteil 34 gezeigt. Dieses Deckelteil 34 weist wiederum Ausbuchtungen 35.1 bis 35.8 auf, welche das Entfernen des Deckelteils 34 von einem nicht gezeigten Unterteil 36 oder des gesamten Aufnahmegehäuses 33 von einem nicht gezeigten Sterilisierbehälterdeckel auf einfache Weise ermöglichen soll.

In Figur 13 ist eine vergrössert dargestellte geschnittene Seitenansicht der Figur 12 an der Schnittlinie A-A nach Figur 12 gezeigt. Das Aufnahmegehäuse 33 ist dafür ausgelegt, dass beispielsweise ein Filter 37 zwischen dem Deckelteil 34 und dem Unterteil 36 geklemmt gehalten werden kann. So dass die in Figur 12 gezeigten Öffnungen 38 direkt mit dem Filter 37 in Wirkverbindung stehen. Dazu wird die durch einen nicht gezeigten Deckel durch Druck in das Innere des Aufnahmegehäuses 33 gebracht, so dass die Sterilisationsluft durch den Filter 37 und die Öffnungen 38 in das Innere des Sterilisierbehälters eingedrückt wird.

In Figur 13 ist wiederum gezeigt, wie ein umlaufender Silikonfilter 39 einen hermetischen Abschluss eines nicht gezeigten Deckelelements bildet. Ausserdem sind zwei Kugelelemente 40, 41 in Figur 13 zu erkennen, welche federgelagert sind und dazu dienen, sich in entsprechende Öffnungen eines Deckelbehälters einzulassen und ein einfacheres Montieren des Aufnahmegehäuses 33 an einem nicht gezeigten Deckel zu erleichtern. Die Kugelelemente 40, 41 sind hierzu federgelagert.

Bei dem Filter 37 kann es sich um einen PTFE- oder Teflon-Filter handeln. Es ist aber auch denkbar, dass es sich um Textil- oder Papierfilter handelt, welche in gleicher Weise eingesetzt werden können. Der modulare Aufbau der Vorrichtung erlaubt ein einfaches Austauschen der verschiedenen Filterarten, in dem durch einfaches Lösen des Deckelteils 34 von dem Unterteil 36 ein anderer Filter klemmend zwischen die beiden Teile 36, 34, eingelegt und verschlossen werden kann. Der Verschluss zwischen dem Deckelteil 34 und dem Unterteil 36 ist derart gestaltet, dass ein Nutzer dies ohne Werkzeug oder nur mit sehr wenig Werkzeug einfach und schnell handhaben kann.

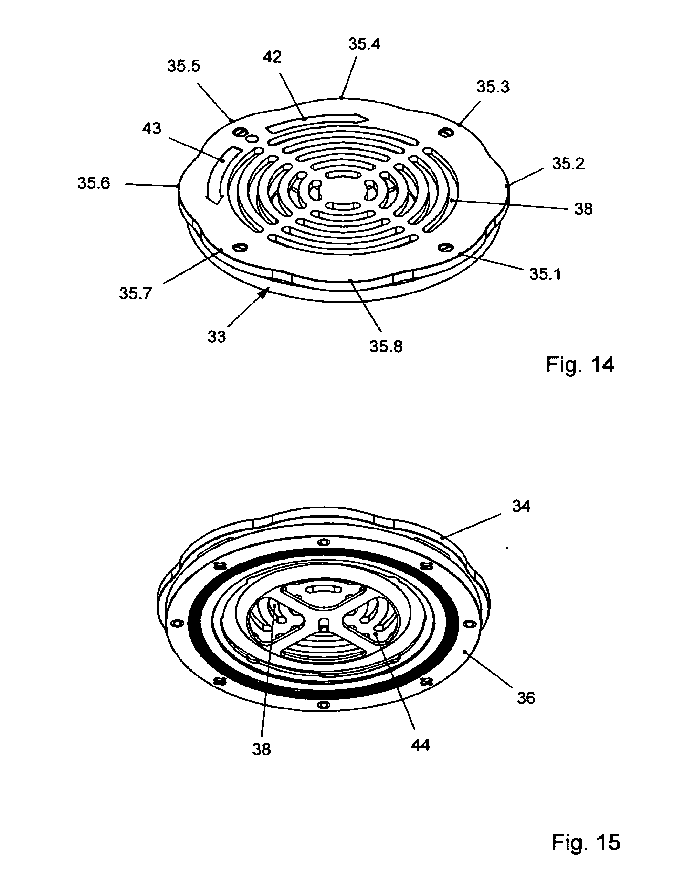

Figur 14 zeigt eine Draufsicht auf das Aufnahmegehäuse nach Figur 12 von schräg oben. Dort sind wieder die Öffnungen 38 sowie die Ausbuchtungen 35.1 bis 35.8 zu erkennen. Ausserdem sind zwei Richtungspfeile 42, 43 zu erkennen, welche dem Nutzer das einfachere Aufbringen des Aufnahmegehäuses 33 auf einen nicht gezeigten Deckel erleichtern soll. Das Aufbringen erfolgt wiederum in der Art, dass auf einen Deckel nach Figur 7 das Aufnahmegehäuse 33 durch Aufsetzen und Entlangführen des Aufnahmegehäuses 33 an den Schraubenelementen 22.1 bis 22.4 bajonettartig an dem Deckel 18 festgelegt werden kann. Das Lösen des Aufnahmegehäuses 33 von dem Deckel 18 erfolgt in umgekehrter Drehrichtung des Aufnahmegehäuses 33.

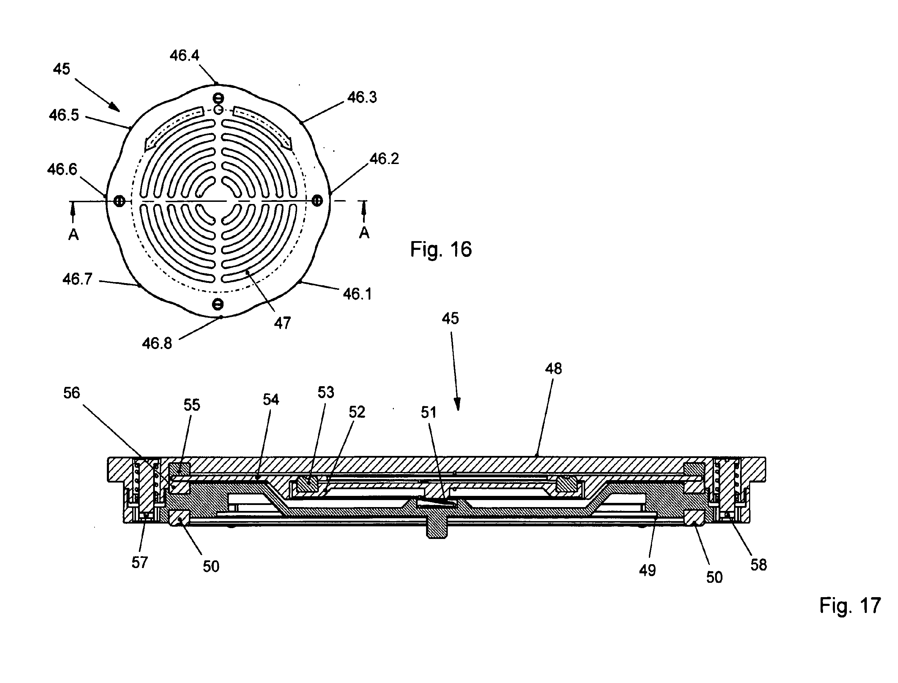

Figur 15 zeigt eine Ansicht der Figur 14 von schräg unten. Dort ist wiederum ein Durchlass 44 gezeigt. Ausserdem sind die Öffnungen 38 zu erkennen, da in dem Ausführungsbeispiel nach Figur 15 kein Filter eingesetzt ist, welcher die Sicht auf die Öffnungen 38 behindern könnte. Figur 16 zeigt wiederum eine Draufsicht auf ein anderes Ausführungsbeispiels eines erfindungsgemässen Aufnahmegehäuses 45. Auch dort sind eine Vielzahl von Ausbuchtungen 46.1 bis 46.8 gezeigt. Ausserdem sind Öffnungen 47 zu erkennen, durch die sterilisierte Luft in das Innere eines Sterilisierbehälters einfliessen kann. Ausserdem ist eine Schnittlinie A-A gezeigt. An dieser Schnittlinie ist Figur 17 entstanden. Figur 17 zeigt, dass das Aufnahmegehäuse 45 wiederum aus einem Deckelteil 48 und einem Unterteil 49 besteht. Das Unterteil 49 weist wiederum eine Silikon-Dichtung 50 auf. Ausserdem ist in der Schnittzeichnung nach Figur 17 eine Druckfeder 51 zu erkennen, welche zwischen einem Zwischenstück 52 und dem Unterteil 49 angebracht ist und die beiden Teile gegeneinander abstützt. Ausserdem weist das Zwischenstück 52 eine umlaufende Silikon-Dichtung 53 auf, welche das Zwischenstück 52 gegen ein Haltestück 54 abdichtet. Das Haltestück 54 wiederum ist derart in dem Aufnahmegehäuse 45 eingelegt, dass es zwischen dem Unterteil 49 und dem Deckelteil 48 gehalten wird. Dies geschieht durch eine Silikon-Dichtung 55, welche mit dem Deckelteil 48 in Wirkverbindung steht und eine Silikon-Dichtung 56, welche mit dem Unterteil in Wirkverbindung steht. Ausserdem sind in der Figur 17 wiederum federgelagerte Kugelelemente 57, 58 gezeigt, welche wiederum ein einfacheres und sicheres Justieren des Aufnahmegehäuses 45 auf einem Deckel 18 gewährleisten sollen. Hierzu fahren die federgelagerten Kugelelemente 57, 58 entlang des Deckels 18, während das Aufnahmegehäuse 45 in den Schraubenelementen 22.1 bis 22.4 eingesetzt und kreisförmige geführt wird, bis bajonettartig ein Verschluss zwischen Aufnahmegehäuse 45 und dem Deckel 18 erreicht ist.

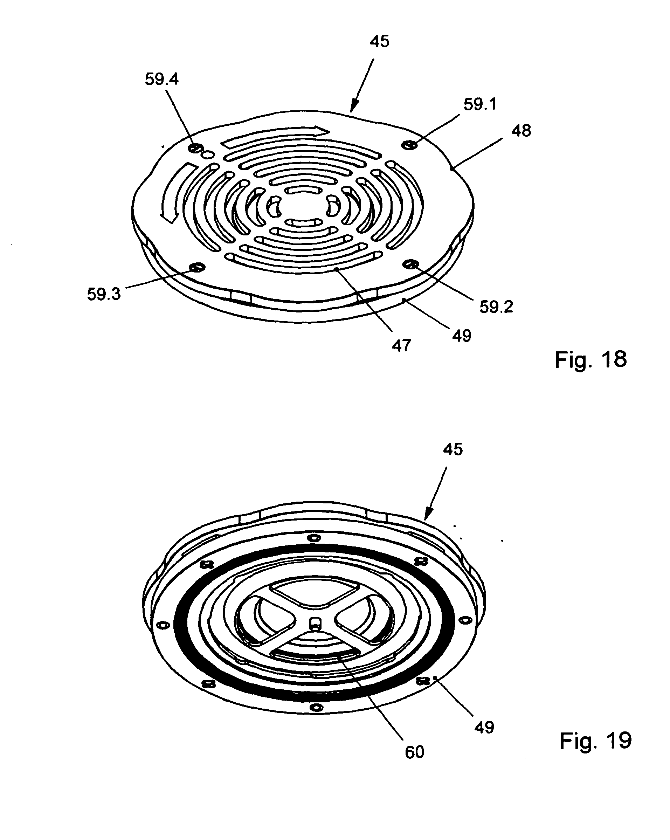

Figur 18 zeigt wiederum eine Draufsicht auf das Aufnahmegehäuse 45 von schräg oben. Dort sind wiederum die Ausbuchtungen 46.1 bis 46.8 sowie die

Öffnungen 47 gezeigt. Daneben ist zu erkennen, wie das Deckelteil 48 und das Unterteil 49 aneinander festliegen. Die Trennung der beiden Teile erfolgt auf einfache Art und Weise durch eine Bajonettfederschraube, wie es auch bei den Aufnahmegehäusen 23, 33 der Fall ist. Die Bajonettfederschrauben 59.1 bis 59.4 sind derart gestaltet, dass ein einfaches Lösen des Deckelteils 48 von dem Unterteil 49 erreicht werden kann.

In Figur 19 ist wiederum gezeigt, wie eine Öffnung 60 des Unterteils 49 vorhanden ist. Nach Aufsetzen des Aufnahmegehäuses auf die Unterseite des Deckels 18 durchströmt die sterilisierende Luft die Öffnung 21 und anschliessend durchtritt es die Öffnung 60 in den Ventilbereich, welcher in erster Linie aus dem Haltestück 54 und dem Zwischenstück 52 besteht. Nach Aufbau eines bestimmten Drucks auf das Zwischenstück 52 wird das Federelement 51 derart verändert, dass ein Spalt zwischen Zwischenstück 52 und Haltestück 54 entsteht und die sterilisierende Luft in das Innere des Sterilisierbehälters einfliessen kann.

**Bezugszeichenliste**

| | | | | | |
|---|---|---|---|---|---|
| 1 | Deckel | 34 | Deckelteil | 67 | |
| 2 | Aufnahmegehäuse | 35 | Ausbuchtung | 68 | |
| 3 | Filter | 36 | Unterteil | 69 | |
| 4 | Verschluss | 37 | Filter | 70 | |
| 5 | Henkel | 38 | Öffnung | 71 | |
| 6 | Öffnung | 39 | Silikonfilter | 72 | |
| 7 | erste Festlege-Löse-Markierung | 40 | Kugelelement | 73 | |
| 8 | Pfeil | 41 | Kugelelement | 74 | |
| 9 | Pfeil | 42 | Richtungspfeil | 75 | |
| 10 | zweite Festlege-Löse-Markierung | 43 | Richtungspfeil | 76 | |
| 11 | Dichtungsring | 44 | | 77 | |
| 12 | Öffnung | 45 | Aufnahmegehäuse | 78 | |
| 13 | Hinterschneidung | 46 | Ausbuchtung | 79 | |
| 14 | Durchbruch | 47 | Öffnung | | |
| 15 | Festlegeprofil | 48 | Deckelteil | | |
| 16 | Anschlag | 49 | Unterteil | i | |
| 17 | Hakenelement | 50 | Silikon-Dichtung | h | |
| 18 | Deckel | 51 | Druckfeder | | |
| 19 | Festlegeprofil | 52 | Zwischenstück | | |
| 20 | Festlegeprofil | 53 | Silikon-Dichtung | | |
| 21 | Durchbruch | 54 | Haltestück | | |
| 22 | Schraubenelement | 55 | Silikon-Dichtung | | |
| 23 | Aufnahmegehäuse | 56 | Silikon-Dichtung | | |
| 24 | Ausbuchtung | 57 | Kugelelement | | |
| 25 | Silikondichtung | 58 | Kugelelement | | |
| 26 | Deckelteil | 59 | Bajonettfederschraube | | |
| 27 | Unterteil | 60 | Öffnung | | |
| 28 | Barrieresystem | 61 | | | |
| 29 | Kugel | 62 | | | |
| 30 | Dichtungspfeil | 63 | | | |
| 31 | Einlass | 64 | | | |
| 32 | Auslass | 65 | | | |
| 33 | Aufnahmegehäuse | 66 | | | |

## Patentansprüche

1. Sterilisierbehälter (S) mit einem Deckel (1) mit Verschluss (4) zur Herstellung einer Verbindung zwischen Sterilisierbehälter (S) und Deckel (1), wobei der Deckel (1) Schraubenelemente (22.1-22.4) zur Anbringung eines Aufnahmegehäuses (23) aufweist,
und die Schraubenelemente (22.1-22.4) derart gestaltet sind, dass das Aufnahmegehäuse (23) ähnlich einem Bajonettverschluss durch Aufsetzen und Drehen wiederlösbar mit dem Deckel (1) verbunden ist, und
das Aufnahmegehäuse (23) Ausbuchtungen (24.1-24.8) aufweist und
ein Filter (3) im Aufnahmegehäuse (23) integriert ist,
wobei
die Schraubenelemente (22.1 - 22.4) den Deckel (1) durchgreifen und eine auf der dem Aufnahmegehäuse (23) gegenuberliegenden Seite des Deckels (1) angeordnete Plastikhaube fest mit dem Deckel (1) verbinden.

2. Sterilisierbehälter (S) nach Anspruch 1, **dadurch gekennzeichnet, dass** der Filter (3) eine Sterilbarriere oder ein Ventilfilter oder ein Teflonfilter ist.

3. Sterilisierbehälter (S) nach Anspruch 1 oder 2, **dadurch gekennzeichnet, dass** das Aufnahmegehäuse (23) auf der dem Deckel (1) zugewandten Seite eine Hinterschneidung (13) aufweist.

4. Sterilisierbehälter (S) nach einem der vorigen Ansprüche, **dadurch gekennzeichnet, dass**
das Aufnahmegehäuse (23) auf der dem Deckel (1) zugewandten Seite einen Dichtungsring (11) umfasst.

5. Sterilisierbehälter (S) nach einem der vorigen Ansprüche, **dadurch gekennzeichnet, dass**
das Aufnahmegehäuse (23) auf der dem Deckel (1) abgewandten Seite eine erste Festlege-Löse-Markierung (7) aufweist.

6. Sterilisierbehälter (S) nach einem der Ansprüche 1 bis 5, **dadurch gekennzeichnet, dass** der im Deckel (1) auf der dem Aufnahmegehäuse (23) zugewandten Seite eine zweite Festlege-Löse-Markierung (10a, 10b) aufweist.

7. Sterilisierbehälter (S) nach Anspruch 5 oder 6, **dadurch gekennzeichnet, dass** die erste Festlege-Löse-Markierung (7) und die zweite Festlege-Löse-Markierung (10a, 10b) zusammenwirken.

8. Sterilisierbehälter (S) nach einem der Ansprüche 1 bis 7, **dadurch gekennzeichnet, dass** der Deckel (1) im mittigen Bereich zwischen dem Aufhahmegehäuse (23) und der Plastikhaube einen Durchbruch (21) mit einer Vielzahl von Öffnungen aufweist.

9. Sterilisierbehälter (S) nach einem der Ansprüche 1 bis 8, **dadurch gekennzeichnet, dass** das Aufhahmegehäuse (23) mindestens eine federgelagerte Kugel (29) aufweist, welche beim Verbinden mit dem Deckel (1) in eine kugelförmige Ausbuchtung des Deckels eingreift.

10. Sterilisierbehälter (S) nach einem der Ansprüche 1 bis 9, **dadurch gekennzeichnet, dass** das Aufnahmegehäuse (23) ein Deckelteil (48) und ein Unterteil (49) aufweist, wobei das Deckelteil (48) und das Unterteil (49) über Bajonettfederschrauben (59.1 - 59.4) miteinander verbunden sind.

11. Sterilisierbehälter (S) nach einem der Ansprüche 1 bis 10, **dadurch gekennzeichnet, dass** das Aufnahmegehäuse (23) zwei Richtungspfeile (42, 43) aufweist.

## Claims

1. Sterilisation container (S) with a lid (1) with closure (4) for producing a connection between sterilisation container (S) and lid (1), wherein the lid (1) possesses screw elements (22.1-22.4) for attaching a receptacle housing (23),
and the screw elements (22.1-22.4) are designed such that the receptacle housing (23) by attaching and turning, similar to a bayonet closure, is releasably connected with the lid (1), and
the receptacle housing (23) possesses bulges (24.1-24.8) and
a filter (3) is integrated in the receptacle housing (23),
wherein
the screw elements (22.1-22.4) reach through the lid (1) and fixedly connect a plastic cover, located on the side of the lid (1) opposite to the receptacle housing (23), with the lid (1).

2. Sterilisation container (S) according to claim 1, **characterised in that** the filter (3) is a sterile barrier or a valve filter or a Teflon filter.

3. Sterilisation container (S) according to claim 1 or 2, **characterised in that** the receptacle housing (23) possesses an undercut (13) on the side facing the lid (1).

4. Sterilisation container (S) according to one of the preceding claims, **characterised in that** the receptacle housing (23) comprises a sealing ring (11) on the side facing the lid (1).

5. Sterilisation container (S) according to one of the preceding claims, **characterised in that**
the receptacle housing (23) possesses a first connect-disconnect marking (7) on the side facing away from the lid (1).

6. Sterilisation container (S) according to one of claims 1 to 5, **characterised in that**
the lid (1) possesses a second connect-disconnect marking (10a, 10b) on the side facing the receptacle housing (23).

7. Sterilisation container (S) according to claim 5 or 6, **characterised in that** the first connect-disconnect marking (7) and the second connect-disconnect marking (10a, 10b) cooperate.

8. Sterilisation container (S) according to one of claims 1 to 7, **characterised in that** the lid (1), in the central area between the receptacle housing (23) and the plastic cover, possesses an aperture with a plurality of openings.

9. Sterilisation container (S) according to one of claims 1 to 8, **characterised in that** the receptacle housing (23) possesses at least one spring-loaded ball (29) that when connected to the lid (1) engages into a spherical indentation of the lid.

10. Sterilisation container (S) according to one of claims 1 to 9, **characterised in that** the receptacle housing (23) possesses a lid part (48) and a lower part (49), wherein the lid part (48) and the lower part (49) are connected together with bayonet coil springs (59.1-59.4).

11. Sterilisation container (S) according to one of claims 1 to 10, **characterised in that** the receptacle housing (23) possesses two directional arrows (42, 43).

## Revendications

1. Récipient de stérilisation (S) comprenant un couvercle (1) avec un système de fermeture (4) servant à établir une liaison entre le récipient de stérilisation (S) et le couvercle (1), sachant que le couvercle (1) présente des éléments à vis (22.1 - 22.4) destinés à la mise en place d'un boîtier de réception (23),
et les éléments à vis (22.1 - 22.4) sont configurés de telle manière que le boîtier de réception (23) est relié au couvercle (1) de manière redétachable en le posant et en le tournant à la manière d'un système de fermeture à baïonnette, et
le boîtier de réception (23) présente des renflements (24.1 - 24.8), et
un filtre (3) est intégré dans le boîtier de réception (23),
sachant que les éléments à vis (22.1 - 22.4) traversent le couvercle (1) et relient de manière solidaire au couvercle (1) une housse en matière plastique disposée sur le côté, faisant face au boîtier de réception (23), du couvercle (1).

2. Récipient de stérilisation (S) selon la revendication 1, **caractérisé en ce que** le filtre (3) est une barrière stérile ou un filtre à soupape ou un filtre en téflon.

3. Récipient de stérilisation (S) selon la revendication 1 ou 2, **caractérisé en ce que** le boîtier de réception (23) présente, sur le côté tourné vers le couvercle (1), une contre-dépouille (13).

4. Récipient de stérilisation (S) selon l'une quelconque des revendications précédentes, **caractérisé en ce que**
le boîtier de réception (23) comprend, sur le côté tourné vers le couvercle (1), une bague d'étanchéité (11).

5. Récipient de stérilisation (S) selon l'une quelconque des revendications précédentes, **caractérisé en ce que**
le boîtier de réception (23) présente, sur le côté opposé au couvercle (1), une première marque de serrage-desserrage (7).

6. Récipient de stérilisation (S) selon l'une quelconque des revendications 1 à 5, **caractérisé en ce que** le couvercle (1) présente, sur le côté tourné vers le boîtier de réception (23), une deuxième marque de serrage-desserrage (10a, 10b).

7. Récipient de stérilisation (S) selon la revendication 5 ou 6, **caractérisé en ce que** la première marque de serrage-desserrage (7) et la deuxième marque de serrage-desserrage (10a, 10b) coopèrent.

8. Récipient de stérilisation (S) selon l'une quelconque des revendications 1 à 7, **caractérisé en ce que** le couvercle (1) présente, dans la zone centrale entre le boîtier de réception (23) et la housse en matière plastique, une percée (21) pourvue d'une pluralité d'orifices.

9. Récipient de stérilisation (S) selon l'une quelconque des revendications 1 à 8, **caractérisé en ce que** le boîtier de réception (23) présente au moins une bille (29) montée sur ressort,
laquelle vient en prise, lors de la liaison au couvercle (1), avec un renflement de forme sphérique du couvercle.

10. Récipient de stérilisation (S) selon l'une quelconque des revendications 1 à 9, **caractérisé en ce que** le boîtier de réception (23) présente une partie couvercle (48) et une partie inférieure (49), sachant que la partie couvercle (48) et la partie inférieure (49) sont reliées l'une à l'autre par l'intermédiaire de vis à ressort à baïonnette (59.1 - 59.4).

11. Récipient de stérilisation (S) selon l'une quelconque des revendications 1 à 10, **caractérisé en ce que** le boîtier de réception (23) présente deux flèches de direction (42, 43).
